# EUROPEAN PATENT APPLICATION

(11) **EP 4 218 593 A1**
(43) Date of publication of application: **02.08.2023**
(21) Application number: 22154218.6
(22) Date of filing: 31.01.2022
(51) Int. Cl.: A61B 8/00, A61B 8/08

(54) **ULTRASOUND SCANHEAD ADAPTER**

(71) Applicant: Cortex Technology Aps, 9560 Hadsund (DK)
(72) Inventor: Schärfe Baltzer-Thomsen, Christian, 9560 Hadsund (DK)
(74) Representative: Høiberg P/S

(57) **Abstract**

The invention regards an ultrasound scanhead adapter for attachment to an ultrasound scanhead, the adapter comprising
- a distal end having a frame section for contacting the skin of a subject, a height of the frame section defining
i. a cavity, and
ii. a fixed distance between the skin surface of the subject and a top of the ultrasound scanhead during scanning, and

- a proximal end forming a reversible snap-fit connection to the scanhead.

## Description

### Technical field

The present disclosure relates to an ultrasound scanhead adapter for an ultrasound scanhead, and use of the adapter and scanhead for ultrasound skin imaging.

### Background

Ultrasonic transducers or probes are used in a variety of non-invasive visualization applications, such as for fetal ultrasound imaging and skin imaging. The imaging is typically performed by moving a scanhead of a hand-held probe over the skin surface of a person, whereby a cross-sectional image of the body below the skin surface may be obtained.

To facilitate more focused and higher resolution imaging, the ultrasonic transducer may include a probe positioning system, such that the acoustic window of the scanhead is fixed at a predetermined distance to the surface of the skin. This way, the focal range of the scanhead is controlled and focused at the depth of the body structure, which is the subject of the ultrasonic examination. For stability, the probe positioning system is typically permanently fastened to the scanhead or rigidly attached during assembling by e.g. glue.

EP1958569 discloses an ultrasound couplant device including a gel pad for focusing the ultrasound beam close to the surface of the skin, where the gel pad may be attached by an adhesive.

EP0527651 discloses a retention member attachable to an ultrasonic scanhead for examining subcutaneous tissue. The retention member defines a volumetric region between the scanhead acoustic window and the subject, where the volumetric region is filled with a gel pad or another medium transmissive of ultrasound. The gel pad is inserted before attaching the retention member to the scanhead, and acoustic coupling is ensured by the gel pad protruding or extending through the distal end of the retention member, such that only the gel pad is in contact with the skin.

US20200138409 discloses a spacer for an ultrasound probe for examining subcutaneous tissue. The spacer is made of a compliant material for example a hydrogel having a concavity. The gel spacer may be attached to a lip frame, e.g. by adhesives, from which it protrudes distally, such that only the gel spacer is in contact with the skin.

### Summary

The present disclosure provides an ultrasound scanhead adapter with an integrated optical positioning system, which facilitate high precision imaging, even at high frequencies and high scanning rates. The adapter is configured to be detachably attached to an ultrasound scanhead by a fast, simple, fail-safe, and flexible fastening mechanism, which simultaneously provide a highly rigid and stable connection when attached to the scanhead, and specifically the fastening mechanism is a manual and reversible snap-on mechanism or snap-fit connection. This facilitates reuse of the scanhead, while discarding the adapter and associated fluids, to reduce the risk of cross-contamination. Thus, optionally the adapter may be disposable and configured for single-use. Alternatively, the adapter may be unsnapped and sterilized for a following ultrasound examination. This way the adapter is also configured for recycling, to reduce industrial waste.

The integrated optical positioning system is based on the adapter's distal end having a frame section for contacting the skin of a subject, where the height of the frame section defines a cavity and a fixed distance between the skin surface of the subject and a top of the fastened ultrasound scanhead during scanning. The cavity is advantageously loaded with an ultrasound transmissive medium, such as a fluid or gel, from the distal end after attachment to a scanhead, such that the assembly and operation of the adapter is simplified and more flexible.

The adapter is found to be particularly suitable for high frequency ultrasound skin imaging, especially for scanning frequencies between 10-50 MHz or 15-50 MHz. For example, surprisingly reproducible and high resolution ultrasound images may be obtained at e.g. 10 MHz or 18 MHz. Particularly, the adapter and frame section is configured such that the high frequency ultrasound beam is accurately and reproducible focused close to the surface of the skin, and thereby provide high resolution ultrasound images of the skin surface and the different layers of the skin. Thus, the adapter may be dimensioned to provide high definition real-time images suitable for estimation of skin tumors, psoriasis, scleroderma, skin ageing, cellulite, efficacy testing of cosmetics and pharmaceutical products, and monitoring the collagen effect of skin rejuvenating procedures, such as non-ablative laser treatment or cosmeceuticals.

A first aspect of the disclosure relates to an ultrasound scanhead for attachment to an ultrasound scanhead, the adapter comprising
- a distal end having a frame section for contacting the skin of a subject, a height of the frame section defining
   i. a cavity, and
   ii. a fixed distance between the skin surface of the subject and a top of the ultrasound scanhead during scanning, and
- a proximal end forming a reversible snap-fit connection to the scanhead.

A second aspect of the disclosure relates to an ultrasound scanhead comprising the adapter according to the first aspect.

A third aspect of the disclosure relates to use of the ultrasound scanhead adapter according to the first aspect or the ultrasound scanhead of the second aspect for ultrasound skin imaging.

### Description of Drawings

The invention will in the following be described in greater detail with reference to the accompanying drawings.
Figure 1 shows an embodiment of the adapter according to the present disclosure in perspective view as seen from (A) the left, and (B) the right.
Figure 2 shows an embodiment of the adapter according to the present disclosure in cross-sectional view as seen from the left and right sides (A-B), the top and bottom (C-D), the proximal rear and the distal front end (E-F).
Figure 3 shows an embodiment of the adapter according to the present disclosure in perspective view, where (A) shows the detached adapter, and (B) shows the adapter fastened to an ultrasound scanhead.
Figure 4 shows an embodiment of the adapter according to the present disclosure in perspective view, where (A) shows the distal front, and (B) the proximal rear end.
Figure 5 shows an embodiment of the adapter according to the present disclosure in perspective view, where (A) shows the distal front, and (B-C) the proximal rear end.
Figure 6 shows an embodiment of the adapter according to the present disclosure in cross-sectional view as seen from the left and right sides (A-B), the top and bottom (C-D), the proximal rear and distal front end (E-F).
Figure 7 shows an embodiment of the adapter according to the present disclosure in perspective view, where (A) shows a detached adapter, (B) shows an adapter fastened to an ultrasound scanhead, and (C) shows the distal front of the fastened adapter shown in (B).
Figure 8 shows embodiments of the adapter according to the present disclosure in cross-sectional view, where (A) and (D) show embodiments of a detached adapter, and (B) and (C) show embodiments of an adapter fastened to an ultrasound scanhead.
Figure 9 shows an embodiment of (A) a hand-held ultrasound scanhead used for high frequency ultrasound skin imaging of facial skin, and (B) an exemplary cross-sectional image which may be used to evaluate pigmented nevus, collagen content, epidermal thickness, dermal tumors, and/or epidermal thickness.
Figure 10 shows embodiments of the adapter according to the present disclosure in cross-sectional view similar to Figure 2, where exemplary dimensions in millimetres are indicated. (A) shows the cross-sectional view as seen from a top side, and (B) shows the cross-sectional view from the proximal rear end.
Figure 11 shows embodiments of the adapter according to the present disclosure in cross-sectional view similar to Figure 2, where exemplary dimensions in millimetres are indicated. (A) shows the cross-sectional view as seen from the left side, and (B) shows the cross-sectional view as seen from the right side.
Figure 12 shows an embodiment of the adapter according to the present disclosure, when fastened to an ultrasound scanhead. (A) shows a perspective view, and (B) shows a cross-sectional view.
Figure 13 shows a close-up on an embodiment of the adapter according to the present disclosure, when fastened to an ultrasound scanhead.

### Detailed description

The invention is described below with the help of the accompanying figures. It would be appreciated by the people skilled in the art that the same feature or component of the device are referred with the same reference numeral in different figures. A list of the reference numbers can be found at the end of the detailed description section.

### Reversible connection

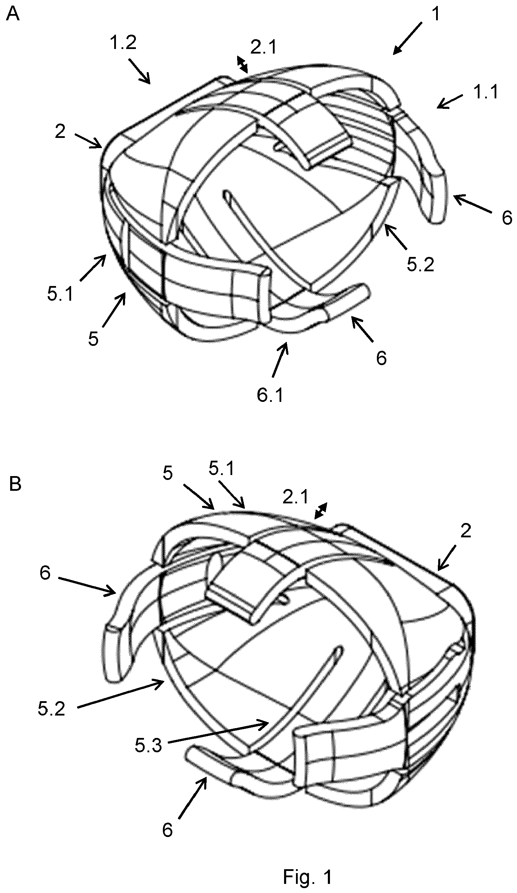

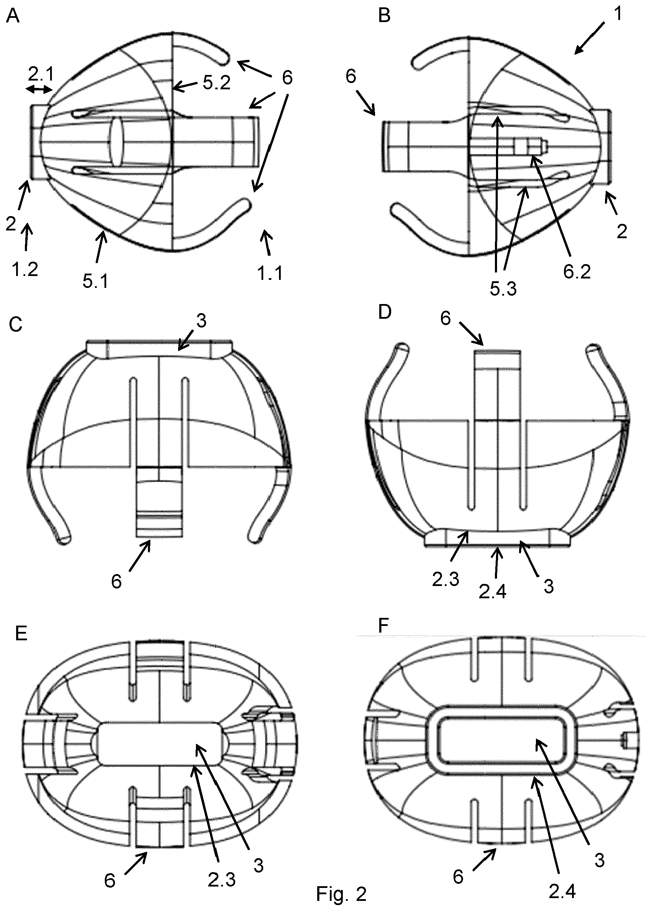

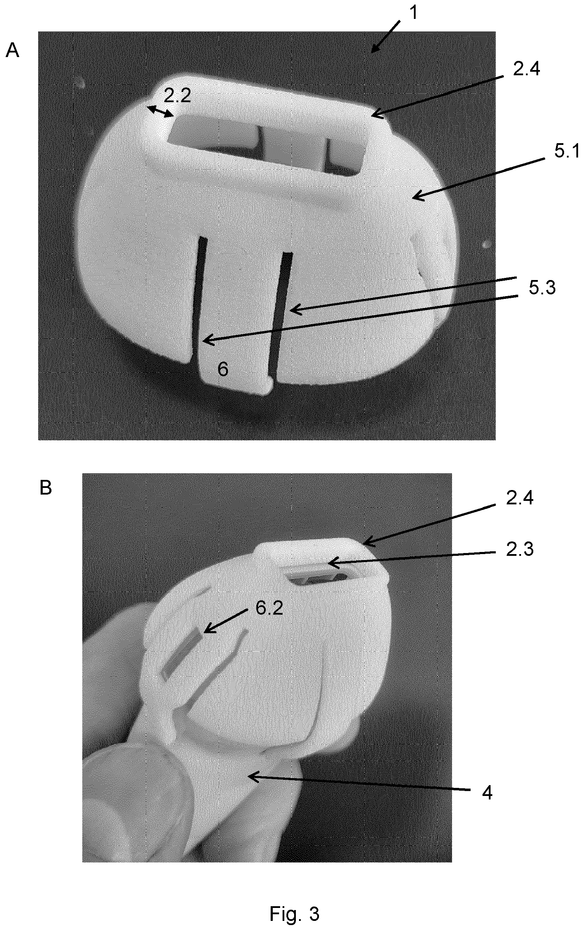

Figures 1 and 2 show an embodiment of the adapter 1 for an ultrasound scanhead according to the present disclosure. Figure 1 shows the adapter in perspective view, and Figure 2 the adapter in cross-sectional view, as seen from the left and right sides (A-B), the top and bottom (C-D), proximal rear and distal front end (E-F), of the adapter as oriented in Figure 1. Figure 3 shows photos of the adapter before (A) and after attachment to an ultrasound scanhead (B).

Figure 1 shows that the adapter **1** has a distal end **1.2** for contacting the body to be examined, e.g. the skin surface of a subject, and a proximal end **1.1** for forming a snap-fit connection to the scanhead **4,** as most clearly seen in Figures 3, 7, and 8. Thus, the adapter may be snap-fitted to an ultrasound scanhead, and then moved/scanned across the skin surface of a subject, to obtain a cross-sectional image of the body below the skin surface. Subsequently, the adapter may be unsnapped and disposed off before the next examination, such that the risk of cross-contamination is reduced.

Hence, the adapter comprises a proximal end for forming a reversible snap-fit connection to the scanhead. This means that the adapter is detachably attached to the scanhead, which allows reusing the scanhead while discarding the adapter, possibly including fluids or gels associated with the adapter, as further described below. Accordingly, the adapter is disposable or configured for single-use. Alternatively, the adapter may be unsnapped and sterilized for a following ultrasound examination. The adapter is therefore also configured for recycling, to reduce industrial waste. This is in contrast to the adapter being assembled to be permanently fastened to, or an integral part of, the scanhead, such that the scanhead and the adapter is a single unit after use.

A reversible snap-fit connection provides a fast, simple, fail-safe, and flexible fastening mechanism or snap-on mechanism. Hence, the adapter may be manually assembled and operated, and then disassembled, such that the adapter is disposable after use and adapted for single-use, or configured for recycling.

In an embodiment of the disclosure, the adapter is disposable and configured for single-use. In an alternative embodiment, the adapter is configured for recycling.

The adapter is advantageously manufactured as a one-piece unit, e.g. by injection molding or 3D printing. Further, the snap-fit feature and the integrated optical positioning system may advantageously be obtained via polymer based plastic materials, which are suitable for injection molding or 3D printing, such as polycarbonates or copolymers, e.g. ABS (acrylonitrile butadiene styrene) copolymers.

In an embodiment of the disclosure, the adapter comprises a polymeric material selected from the group of: polycarbonates, polystyrenes, polyamides, polyurethanes, polyethylene, and copolymers, such as ABS copolymers. In a further embodiment, the adapter is obtained by injection molding.

The reversible snap-fit connection is further configured to form a mechanical stable connection to the scanhead. Specifically, the snap-fit connection is configured to provide a surprisingly rigidly fixed and stable connection when attached to the scanhead, which ensures that the adapter cannot be displaced relative to the scanhead when attached, such as displaced by translation or rotation. The mechanically stable connection may specifically be obtained by a snap-fit connection based on a polymeric material with sufficiently high stiffness combined with sufficient high toughness, which will allow a rigid and stable abutting communication between the adapter and the scanhead when attached, simultaneously with a sufficiently flexible and robust snap-fit during attachment/detachment, with reduced risk of fracturing the snap-fit mechanism.

The mechanism of a snap-fit is based on a structure, such as a cantilever or a tab, having two configurations: a relaxed, non-deflected configuration, and a mechanically stressed, deflected configuration. The stressed configuration occurs when the tab is attached in a first end, and then the second end is passed and elastically deflected around a protrusion. After passing the protrusion the second end is relaxed into a non-deflected configuration, and will be locked towards the protrusion. The tab is typically an elongated flex member, also referred to as a tongue or a finger, and may also have an annular or partly annular shape. It follows that the mechanical properties of the adapter will determine the flexibility, strength, robustness, and stability of the snap-fit. For example, the more brittle the material of the adapter, then the tab will be less robust and more prone to fracture in the deflected configuration. Also, the lower the stiffness or flexural strength of the material of the adapter, the less stable the connection.

Preferably, the adapter is based on a polymeric material which is less brittle, and more tough than polycarbonate, while having sufficient stiffness and yield. For example, the adapter is advantageously made of Novodurs, such as Novodur HD M203FC.

Advantageously, the adapter comprises or is made of a polymeric material having a toughness at 23° of between 10-30 kJ/m², measured as notched impact strength (Charpy) according to ISO 179/1eA, and/or an impact strength at 23° of between 80-130 kJ/m², measured as Charpy according to ISO 179/1eU.

In an embodiment of the disclosure, the adapter comprises a polymeric material having a notched impact strength at 23° of between 10-30 kJ/m², more preferably between 12-25 kJ/m², and most preferably between 14-20 kJ/m², such as 15 or 18 kJ/m², and/or an impact strength at 23° of between 80-130 kJ/m², more preferably between 90-120 kJ/m², and most preferably between 100-120 kJ/m², such as 110 kJ/m².

Advantageously, the adapter comprises a polymeric material having a specific range of tensile modulus, and/or yield stress, and/or yield strain, as measured by ISO 527.

In an embodiment of the disclosure, the adapter comprises a polymeric material having a tensile modulus of between 2000-3000 MPa, more preferably between 2200-2800 MPa, and most preferably between 2300-2600 MPa, such as 2400 or 2500 MPa. In a further embodiment, the adapter comprises a polymeric material having a yield stress of between 20-80 MPa, more preferably between 30-70 MPa, and most preferably between 40-60 MPa, such as 46, 50, 54 MPa. In a further embodiment, the adapter comprises a polymeric material having a yield strain of between 0.5-10%, more preferably between 1-7%, and most preferably between 1.5-5%, such as 2, 2.6, or 3%.

Furthermore, the polymeric material may have a stiffness or flexural strength at 23° of between 40-200 MPa, as measured according to ISO 178.

In an embodiment of the disclosure, the adapter comprises a polymeric material having a flexural strength at 23° of between 40-200 MPa, more preferably between 50-150 MPa, and most preferably between 60-100 MPa, such as 80 MPa.

### Integrated optical positioning system

As described above, the adapter is reversibly snap-fitted to the ultrasound scanhead, and then moved/scanned across the skin surface of a subject, to obtain a cross-sectional image of the body below the skin surface. The adapter comprises an integrated optical positioning system, which ensures a predefined and fixed distance between the skin surface of the subject and the attached ultrasound scanhead, such that the ultrasound beam from the scanhead is focused at the skin surface and the layers of the skin. By the term "integrated" is meant that the distance between the skin surface and the scanhead is inherently determined by the adapter and the adapter structural geometry, and not by auxiliary parts, such as a gel that is applied. This way, the adapter facilitates high definition images of the skin surface and skin layers.

The predefined and fixed distance between the skin surface and the scanhead is obtained by a frame section **2** having a frame section height **2.1,** as seen in Figures 1 and 2A-B. Thus, the frame section acts as an integrated spacer. The frame section may also be referred to as a framed opening or as a window frame, which frames a cavity **3,** as most clearly seen in Figures 2E-F.

The frame section **2** comprises a proximal perimeter **2.3** defining a proximal opening, which is configured to be abutting the scanhead **4,** when the scanhead is snap-fitted into the adapter, as shown in Figures 2 and 3B. The opposite distal perimeter **2.4** of the frame section defines a distal opening in direct contact with the surroundings and is configured for contacting the skin surface of a subject, as seen from Figures 2 and 3B.

Thus, the frame section height **2.1** defines a cavity **3** and a fixed distance between the skin surface of the subject and a top of the attached ultrasound scanhead during scanning. The cavity is advantageously loaded with an ultrasound transmissive medium, such as a couplant gel. Advantageously, the couplant gel is loaded from the distal end **1.2** via the distal opening defined by the distal perimeter **2.4,** after the scanhead is attached, as shown in Figure 3B. For example, the gel may be applied into the cavity **3** in Figure 3, and excess gel may be scraped off by a scraping tool levelling the gel layer to be flush with the frame section height, e.g. such that a gel layer of 1 mm is obtained within the cavity. Since the couplant gel is associated with the adapter cavity, the couplant gel may be easily disposed off simultaneously with the single-use adapter.

The adapter and frame section height **2.1** may be calibrated according to the focal length of the focused ultrasound scanhead, such that the specific ultrasound beam is focused at the skin surface depth or a specific skin layer depth. Preferably, the frame section is adapted for high frequency ultrasound skin surface and skin layer imaging, particularly for scanning frequencies between 10-50 MHz or 15-50 MHz, such as 18 MHz. Thus, the adapter is particularly suitable for estimation of skin tumors, psoriasis, scleroderma, skin ageing, cellulite, efficacy testing of cosmetics and pharmaceutical products, and monitoring the collagen effect of skin rejuvenating procedures, such as non-ablative laser treatment or cosmeceuticals.

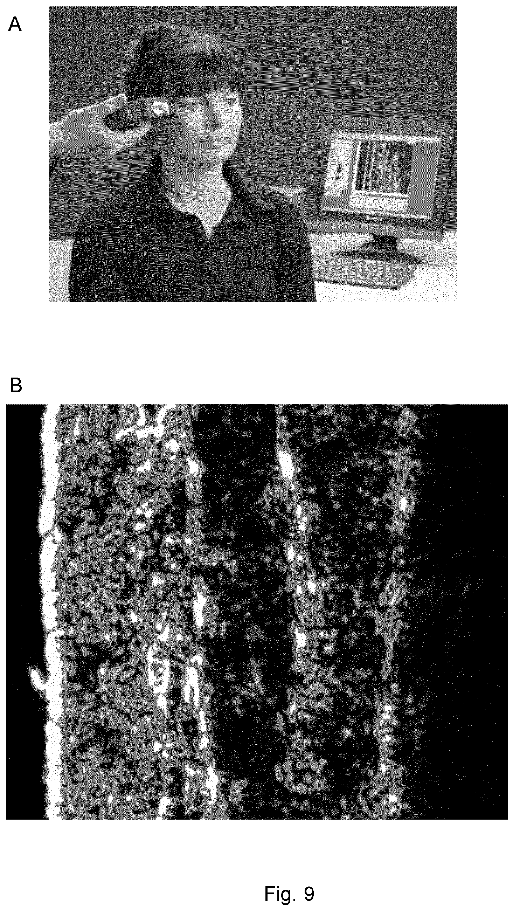

Figure 9 shows an embodiment of (A) a hand-held ultrasound scanhead **4** used for high frequency ultrasound skin imaging of facial skin, and (B) an exemplary cross-sectional image of different layers of skin, which may be used to evaluate pigmented nevus, collagen content, epidermal thickness, dermal tumors, and/or epidermal thickness.

It follows that the disposable adapter the adapter may be applied for stationary as well as for compact and portable scanheads.

### Adapter proximal end

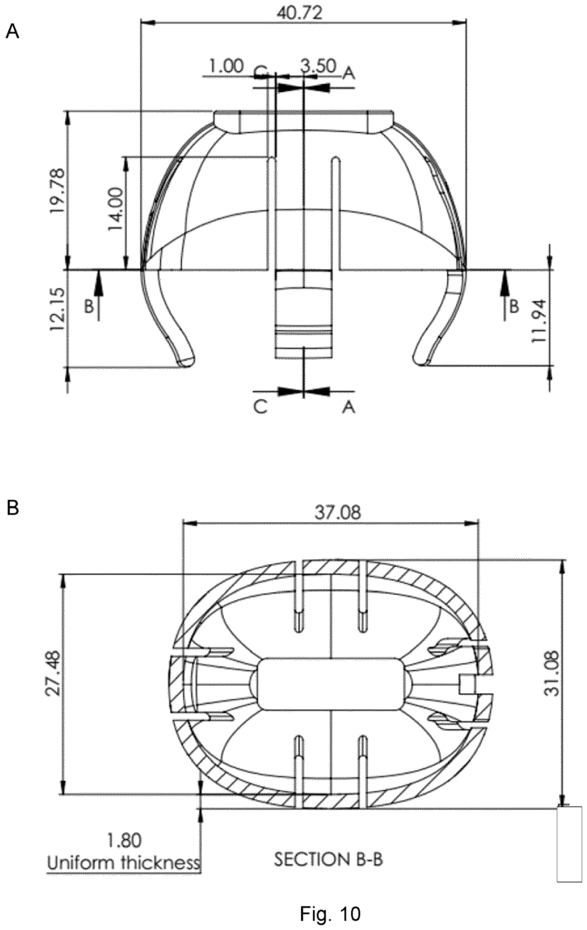

The proximal end **1.1** of the adapter is configured to form a mechanical stable connection to the scanhead **4,** as seen from Figures 3, 7 and 8, showing the detached adapter and the attached adapter. A rigidly fixed and mechanical stable connection may be obtained by a snap-on mechanism, such as a snap-fit connection, which thereby ensures a mechanical rigid and stable position of the adapter relative to the scanhead. It follows that the mechanical stability of the snap-fit connection will be determined by the adapter material, as well as the geometrical shape and dimensions of the snap-fit. Figures 10-12 show embodiments of the adapter according to the present disclosure in cross-sectional view similar to Figure 2, where exemplary dimensions in millimetres are indicated.

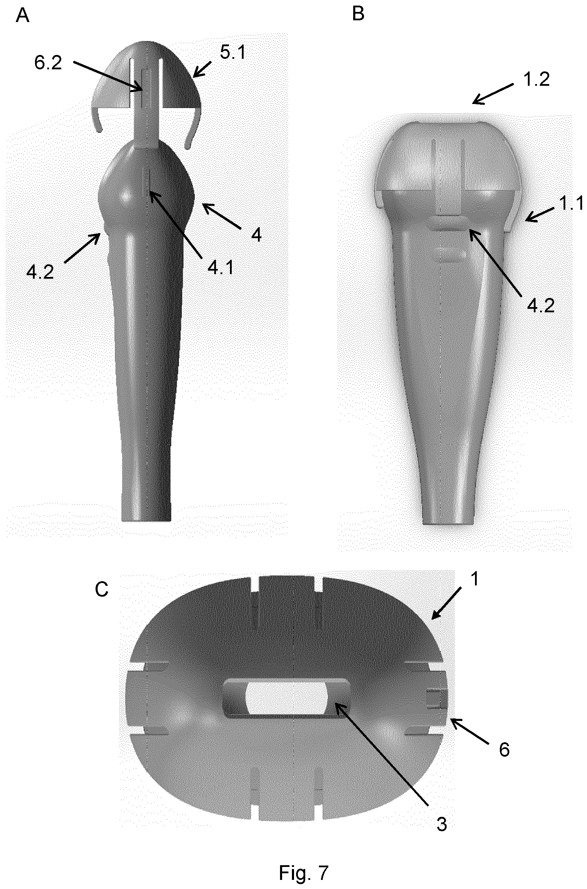

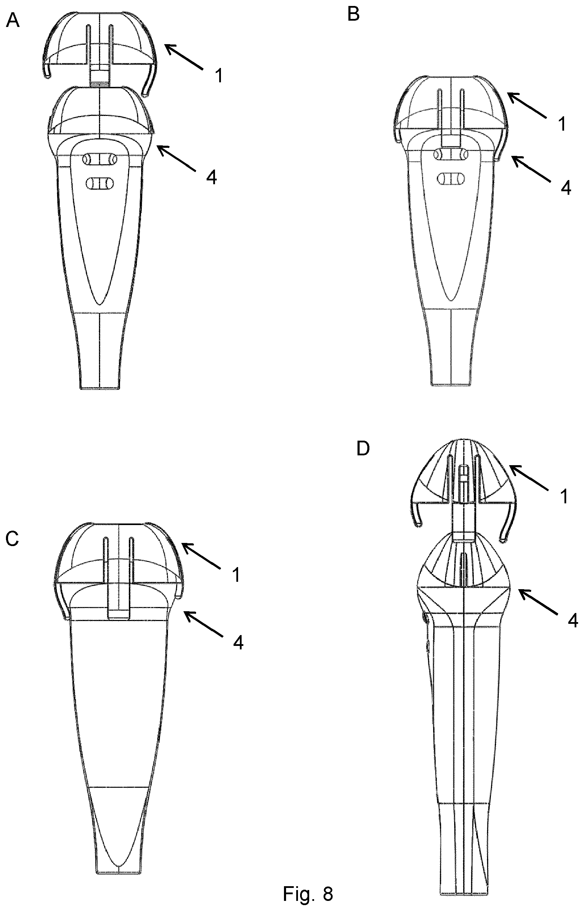

Advantageously, the snap-fit connection is reversible, preferably manually reversible, such that the adapter is easily unsnapped from the scanhead after scanning by finger forces. A reversible snap-fit connection may be obtained by the proximal end **1.1** being configured for partially enclosing or surrounding at least a part of the scanhead. Preferably, the proximal end and scanhead forms abutting matching structures, such as smoothly matching or nested structures, such that the force needed to obtain the snap-fit as well as unsnap is reduced. For example, the proximal end **1.1.** and scanhead **4** may be dome shaped, as seen in Figures 7A, 8A, 8D. The matching dome shaped structures may be attached and detached with manual forces, as indicated in Figures 7 and 8.

The dome shaped proximal end **1.1** further has the advantage that the angular position of the adapter relative to the scanhead is flexible. Hence, the proximal end may be fixed to the scanhead at a predetermined angular position, or the proximal end may be rotation symmetric, such that the proximal end may be fixed to the scanhead at any angular position, and/or rotated relative to the attached scanhead. Preferably, the adapter is configured to be attached to the scanhead in a predetermined arrangement.

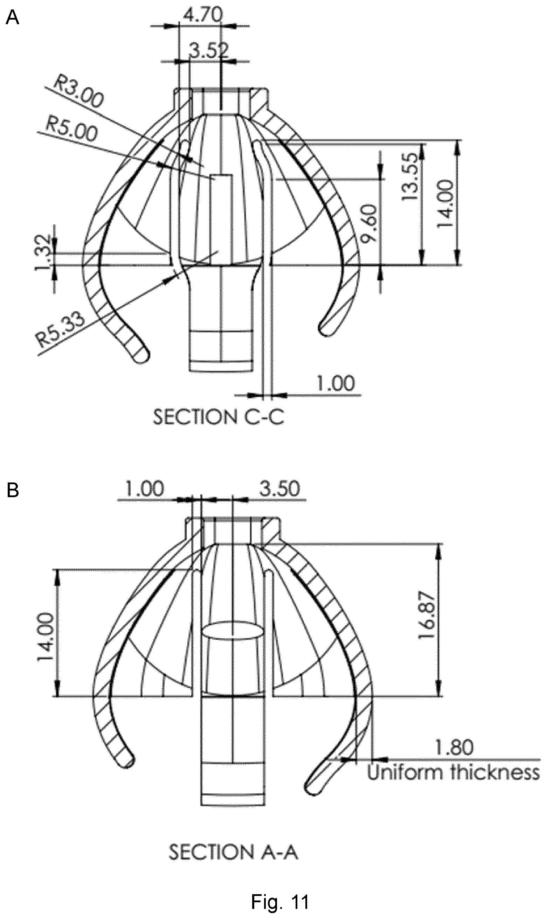

The dome shaped proximal end **1.1** may also be referred to as a chamber **5** defined by the chamber walls **5.1** adapted to abut and enclose/surround an attached scanhead **4.** The chamber is further defined by a proximal opening **5.2** adapted for slidingly receiving the scanhead, such that after attachment of the scanhead, the ultrasound scanhead and/or shaft extends through the proximal opening, as seen in e.g. Figures 1, 2 and 7. It was seen that a specifically stable snap-fit connection, which is also manually reversible, may be obtained with a dome shaped chamber, which preferably has a wall thickness of between 0.5-4 mm, such as 0.5 or 1.80 mm, and further preferably the wall thickness is uniform, as illustrated in Figures 11-12.

In an embodiment of the disclosure, the proximal end is configured for partially enclosing at least a part of the scanhead. In a further embodiment, the proximal end forms a chamber having a chamber wall and a proximal opening adapted for slidingly receiving the scanhead. In a further embodiment, the proximal end forms a dome shaped chamber. In a further embodiment, the chamber wall has a thickness of between 0.2-4 mm, more preferably between 0.4-3 mm, and most preferably between 0.5-2 mm, such as 1.8 mm.

To further reduce the force needed to form the snap-fit and unsnap the adapter from the scanhead, it is advantageous that at least a part of the chamber **5** is elastically deformable or deflectable to facilitate the snap-fit connection. Preferably, the chamber is elastically deformable such that the proximal opening **5.2** may be elastically dilated or expanded. This may for example be obtained by the chamber wall comprising one or more slits **5.3** extending from the proximal opening towards the distal end of the adapter, as indicated in Figures 1-3 and 7. To provide a sufficiently flexible deflection and a sufficiently mechanical stable snap-fit connection, it was found advantageous that the length of the slits was comparable to the height of the chamber, where the height of the chamber is defined as the distance from the chamber proximal opening to the distal end of the adapter. Preferably, the length of the slits is 50-95% of the chamber height, more preferably 60-90%, and most preferably 70-85%, such as 83%. For example, the slits may have a length of between 5-20 mm, such as 14 mm, for an adapter having a chamber height of below 20 mm, such as 16.87 mm, as illustrated in Figure 11B.

In an embodiment of the disclosure, the chamber proximal opening is adapted to be elastically dilated. In a further embodiment, the chamber wall comprises one or more slits extending from the proximal opening towards the distal end. In a further embodiment, the length of the slits is 50-95% of the chamber height, more preferably 60-90%, and most preferably 70-85%, such as 83%. In a further embodiment, the slits have a length of between 5-20 mm, more preferably between 10-18 mm, and most preferably 12-16 mm, such as 14 mm.

Advantageously, the chamber wall comprises more than one slit, thereby facilitating a more elastically dilatable and user friendly adapter. To ensure a simultaneously sufficient mechanical stable snap-fit connection, the two or more slits **5.3** are advantageously located in parallel pairs separated by a predetermined distance, such that a pair of slits define an elongated flex member **6,** also referred to as a cantilever or finger, having a width corresponding to the distance between the slits, as shown in e.g. Figures 1 and 7. To further reduce the force needed to form the snap-fit and unsnap the adapter from the scanhead, the elongated flex member preferably has a width below 2 cm, such as a width of ca. 7 mm, as illustrated in Figure 11A.

In an embodiment of the disclosure, the chamber wall comprises two or more slits in parallel thereby forming an elongated flex member. In a further embodiment, the elongated flex member has a width of between 3-20 mm, more preferably between 4-15 mm, and most preferably between 5-10 mm, such as ca. 6, 7, 8, 9, or 10 mm.

To further reduce the force needed to form the snap-fit and unsnap the adapter from the scanhead, the elongated flex member **6** advantageously has a proximal end adapted to snap-fit over the scanhead, such that the reversible snap-fit connection is primarily obtained via the elongated flex member. For example, the proximal end may include a concavely curved flange **6.1** abutting a further part of a dome shaped scanhead, as shown in most clearly in Figure 7A. Further advantageously, the flange comprises two sections, a first section adapted for snap-fitting over the scanhead, and a second section forming a protrusion or lip for interaction with a finger to facilitate unsnapping, as shown in Figure 3B

In an embodiment of the disclosure, the proximal end of the elongated flex member is configured for elastically snap-fitting over at least a part of the scanhead. In a further embodiment, the proximal end of the elongated flex member comprises a concave flange. In a further embodiment, the elongated flex member comprises a protrusion configured for interaction with a finger.

To ensure a stable snap-fit connection, which at the same time is reversible, the chamber wall advantageously comprises more than two elongated flex members **6,** such as for example four elongated flex members as shown in e.g. Figures 1 and 3. Further mechanically advantageously, the elongated flex members are located at opposite sides of the chamber wall, as shown in Figure 1, and/or at least rotation symmetrically within the chamber wall.

In an embodiment of the disclosure, the chamber wall comprises two or more elongated flex members, such as three, four, or five flex members. In a further embodiment, the two or more elongated flex members are located rotation symmetrically within the chamber wall.

To obtain a more user friendly adapter, the reversible snap-fit connection is primarily obtained via the proximal end of the elongated flex members **6,** and particularly the concave flange **6.1.** The flange may extend proximally to the proximal opening **5.2** of the chamber, as e.g. shown in Figure 1, or the flange may be essentially flush with the proximal opening, as shown in Figure 3A.

In an embodiment of the disclosure, the elongated flex members extend proximally to the proximal opening of the chamber.

To provide a sufficiently flexible deflection and a sufficiently rigid and mechanical stable snap-fit connection, it was found advantageous that the length of the elongated flex members was between 10-40 mm, such as ca. 26 mm as illustrated in Figure 10A.

In an embodiment of the disclosure, the elongated flex members have a length of between 10-40 mm, more preferably between 13-30 mm, and most preferably between 15-28 mm, such as 20, 24, or 26 mm.

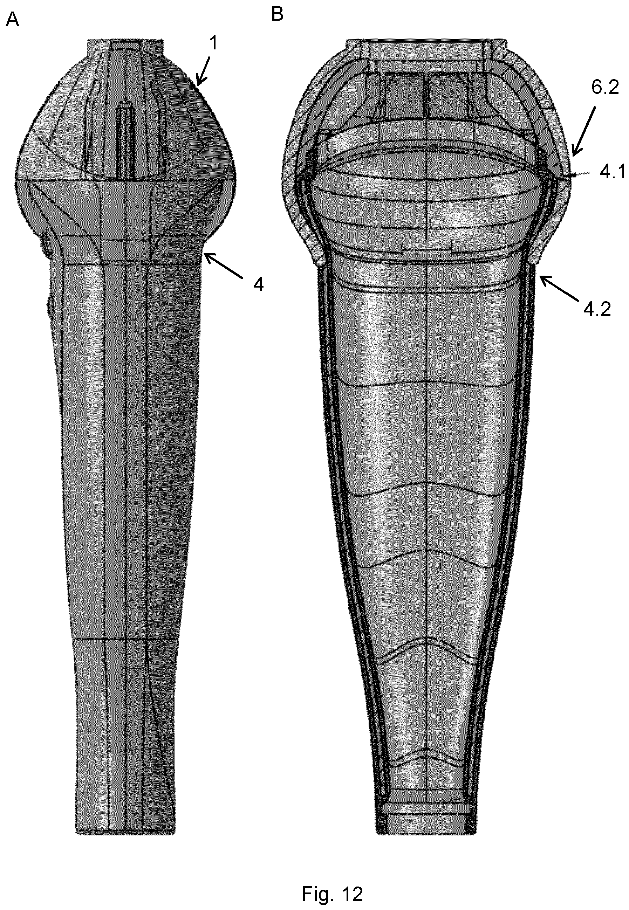

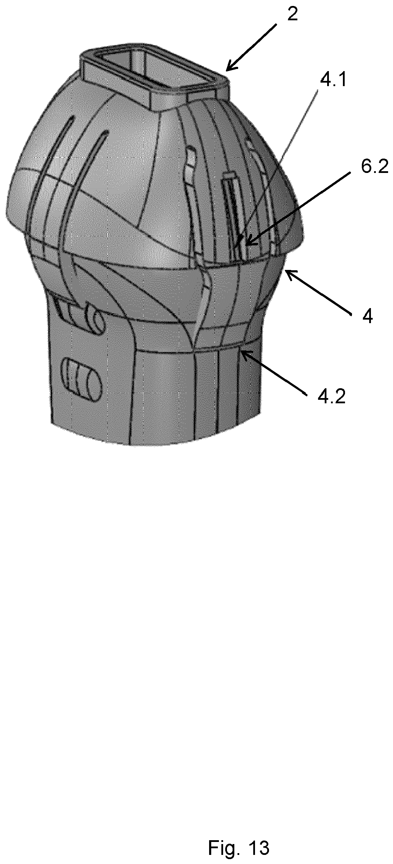

To further increase the mechanical stability of the snap-fit, and to reduce the risk of incomplete attachment between the adapter and the scanhead, it was found advantageous that at least one of the elongated flex members forms an audible snap-fit connection and/or a tactile snap to the scanhead. Thus, the material and dimensions of the at least one elongated flex member is configured to provide a tactile snap and/or audible snap, when the flex member goes from the deflected configuration to the fully relaxed configuration. An audible snap may be obtained if one or more of the elongated flex members comprises an aperture **6.2** as indicated in Figures 2B, 3B, 7A. The aperture is configured to engage or match with a corresponding protrusion **4.1** of the scanhead, as shown in Figure 7A. Preferable the scanhead protrusion is a scanhead barb **4.1,** which forms a barbed engagement with the aperture, thereby providing a significant audible snap-fit. The aperture **6.2** further facilitates that the adapter may be fixed to the scanhead at a predetermined angular position, such that the adapter cannot be rotated relative to the attached scanhead, thereby providing a fail-safe predetermined attachment arrangement. The mechanical stability, and the risk of incomplete attachment may further be improved by the presence of scanhead abutments **4.2,** as shown in Figure 7A. On complete snap-fit connection, the proximal ends of the adapter chamber and/or the elongated flex members are abutting the scanhead abutments **4.2,** as seen in Figure 7B. The aperture **6.2** of the adapter, and the engagement with the scanhead barb **4.1** and scanhead abutment **4.2** is further illustrated in Figures 12-13. To further improve the audible and tactile snap-fit, the aperture has a length comparable or slightly shorter than the slits, such as 9.6 mm, as illustrated in Figure 11A.

In an embodiment of the disclosure, the at least one elongated flex member forms an audible snap-fit to the scanhead. In a further embodiment, the at least one elongated flex member comprises an aperture configured for engaging with a protrusion of the scanhead.

Improved mechanical stability of the snap-fit, and a reduced risk of cracks in the deflected configuration, may be obtained if the at least one elongated flex member has different widths along the longitudinal direction. Advantageously, the elongated flex member comprises a wider section along its length, and preferably the wider section is the section comprising the aperture, as illustrated in Figure 11A. For example, the wider section may be ca. 9.4 mm, compared to ca. 7 mm, as shown in Figure 11A.

In an embodiment of the disclousre, the width of the at least one elongated flex member is wider in the section comprising the aperture.

### Adapter distal end

The distal end **1.2** of the adapter comprises a frame section **2** defining the cavity **3** between the skin surface of the subject and a top of the attached ultrasound scanhead. The frame section may form a distal protrusion, i.e. protruding distally to the chamber **5,** as shown in Figures 1-3.

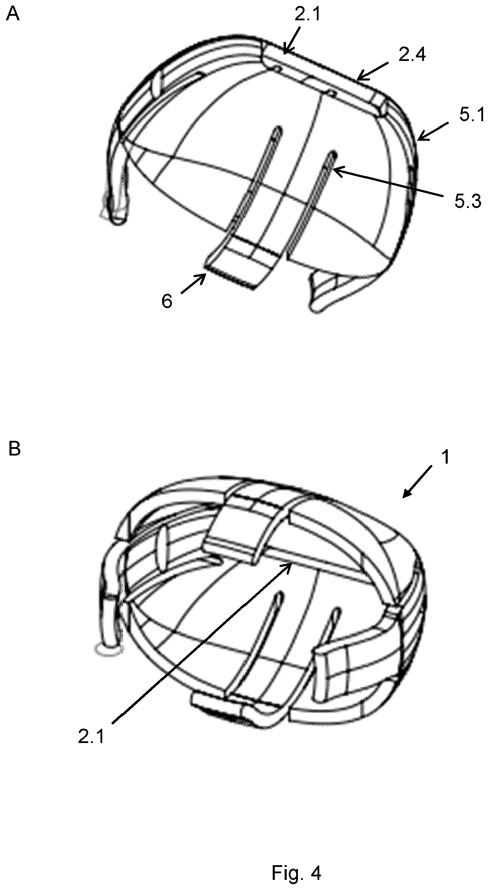

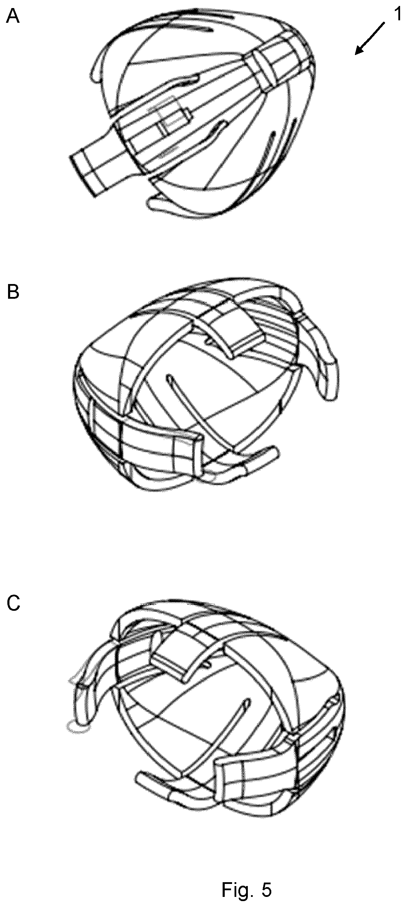

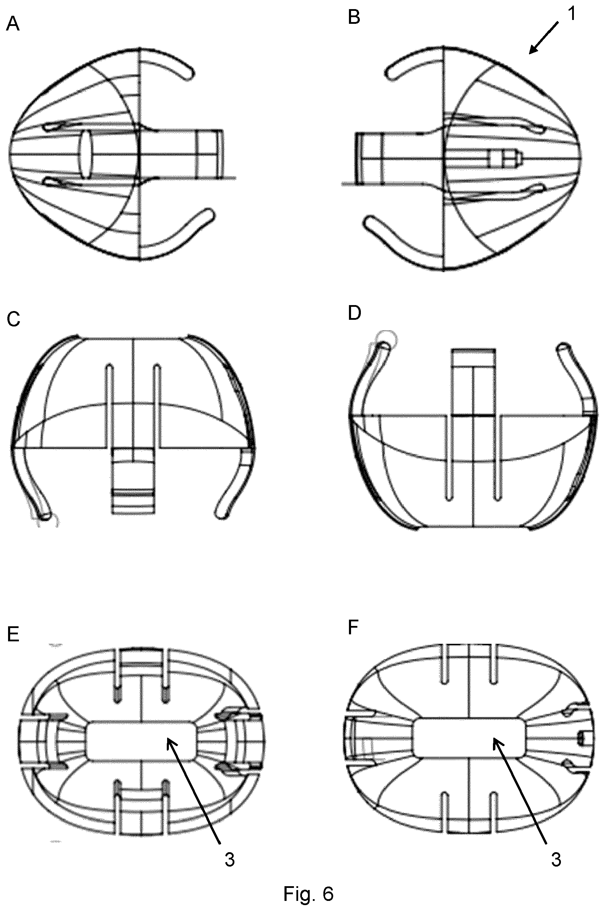

Alternatively, the frame section **2** forms a distal intrusion, i.e. the distal perimeter **2.4** of the frame section is flush with the distal end of the chamber and forms the distal end of the chamber **5,** as illustrated in Figures 4-8. Hence, the frame section height **2.1** may correspond to a frame section depth, and be defined by the chamber wall thickness, as indicated in Figure 4A. An embodiment of an adapter with a cavity **3** defined by a distal intrusion, and attached to a scanhead **4,** is shown in Figure 7C.

In an embodiment of the disclosure, the frame section forms a distal protrusion. In an alternative embodiment, the frame section forms a distal intrusion.

The scanhead and adapter may exert a downward force on the skin surface of the subject, and the pressure from the adapter may affect the skin as well as affect the actual distance between the skin surface and scanhead. Thus, to reduce the potential forces exerted and to provide sharper and real-time images of the skin, the frame section **2** advantageously forms a distal perimeter **2.4** for contacting the skin of the subject, having a regular geometrical shape, such as a rectangular shape, as e.g. seen in Figure 3.

In an embodiment of the disclosure, the frame section forms a distal perimeter for contacting the skin of the subject. In a further embodiment, the perimeter has a shape selected from the group of a: circle, oval, triangular, square, rectangle, rhombus, trapezium, parallelogram, and polygons with more than four sides.

Alternatively, or in addition, the frame section **2** and distal end **1.2** of the adapter may have the shape of a smooth cone, where the smooth cone is contacting the skin surface of the subject, as seen in Figures 7A and 8D. This may further reduce the pressure exerted on the skin by the adapter, and reduce the risk of optical artefacts and unfocused imaging.

In an embodiment of the disclosure, the frame section forms a smooth cone for contacting the skin of the subject.

The frame section **2** may have dimensions configured for reducing the exerted forces on the skin. For example, the height **2.1** of the frame section is preferably between 1-10 mm, and further preferably the width **2.2** of the frame section is similar, e.g. between 1-2 mm, such as ca. 1.2 mm as illustrated in Figure 11A. Thus, the frame section be squared in a cross-sectional view. Further, for simple manufacturing, the frame section height may be identical to the chamber wall thickness.

In an embodiment of the disclosure, the height of the frame section is between 0.5-10 mm, more preferably between 1-8 mm, and most preferably between 1.5-5 mm, such as 2 mm. In a further embodiment, the width of the frame section is between 0.5-10 mm, more preferably between 1-5 mm, and most preferably between 1-2 mm, such as 1.2 mm. In a further embodiment, the height of the frame section is identical to the chamber wall thickness.

The cavity **3** is advantageously loaded with a fluid such as a couplant gel after the scanhead is attached. Thus, gel may be applied or poured from a distal opening and into the protruding cavity in Figure 3B or the intruding cavity in Figure 7C. Subsequently, any excess gel is scraped off such that a gel layer is formed which is flush with the distal perimer **2.4.** This has the advantage that the couplant gel is mainly associated with the adapter cavity, and the couplant gel may hence easily be disposed off simultaneously with the unsnapped single-use adapter

In an embodiment of the disclosure, the frame section forms a distal opening to the cavity. In a further embodiment, the cavity is configured to be loaded with a fluid from the distal end.

### Reference numbers

- 1 -: Adapter
- 1.1 -: Proximal end
- 1.2 -: Distal end
- 2 -: Frame section
- 2.1 -: Frame section height
- 2.2 -: Frame section width
- 2.3 -: Proximal perimeter
- 2.4 -: Distal perimeter
- 3 -: Cavity
- 4 -: Scanhead
- 4.1 -: Scanhead barb
- 4.2 -: Scanhead abutment
- 5 -: Chamber
- 5.1 -: Chamber wall
- 5.2 -: Chamber proximal opening
- 5.3 -: Slit
- 6 -: Elongated flex member
- 6.1 -: Flange
- 6.2 -: Aperture

### Items

The presently disclosed may be described in further detail with reference to the following items.
1. An ultrasound scanhead adapter for attachment to an ultrasound scanhead, the adapter comprising
   - a distal end having a frame section for contacting the skin of a subject, a height of the frame section defining
      i. a cavity, and
      ii. a fixed distance between the skin surface of the subject and a top of the ultrasound scanhead during scanning, and
   - a proximal end forming a reversible snap-fit connection to the scanhead.
2. The adapter according to item 1, wherein the proximal end is configured for partially enclosing at least a part of the scanhead.
3. The adapter according to any of the preceding items, wherein the proximal end forms a chamber having a chamber wall and a proximal opening adapted for slidingly receiving the scanhead.
4. The adapter according to item 3, wherein the proximal end forms a dome shaped chamber.
5. The adapter according to any of items 3-4, wherein the chamber proximal opening is adapted to be elastically dilated.
6. The adapter according to any of items 3-5, wherein the chamber wall has a thickness of between 0.2-4 mm, more preferably between 0.4-3 mm, and most preferably between 0.5-2 mm, such as 1.8 mm.
7. The adapter according to any of items 3-6, wherein the chamber wall comprises one or more slits extending from the proximal opening towards the distal end.
8. The adapter according to item 7, wherein the length of the slits is 50-95% of the chamber height, more preferably 60-90%, and most preferably 70-85%, such as 83%, and/or wherein the slits have a length of between 5-20 mm, more preferably between 10-18 mm, and most preferably 12-16 mm, such as 14 mm.
9. The adapter according to any of items 7-8, wherein the chamber wall comprises two or more slits in parallel thereby forming an elongated flex member.
10. The adapter according to item 9, wherein the elongated flex member has a width of between 3-20 mm, more preferably between 4-15 mm, and most preferably between 5-10 mm, such as 6, 7, 8, 9, or 10 mm.
11. The adapter according to any of items 9-10, wherein the proximal end of the elongated flex member is configured for elastically snap-fitting over at least a part of the scanhead.
12. The adapter according to item 11, wherein the proximal end of the elongated flex member comprises a concave flange.
13. The adapter according to item 12, wherein the flange comprises a protrusion configured for interaction with a finger.
14. The adapter according to any of items 9-13, wherein the chamber wall comprises two or more elongated flex members, such as three, four, or five flex members.
15. The adapter according to item 14, wherein the two or more elongated flex members are located at opposite sides of the chamber wall, and/or rotation symmetrically within the chamber wall.
16. The adapter according to any of items 9-15, wherein the elongated flex members extend proximally to the proximal opening of the chamber.
17. The adapter according to any of items 9-16, wherein the elongated flex members have a length of between 10-40 mm, more preferably between 13-30 mm, and most preferably between 15-28 mm, such as 20, 24, or 26 mm.
18. The adapter according to any of items 9-16, wherein the at least one elongated flex member forms an audible snap-fit to the scanhead.
19. The adapter according to item 18, wherein the at least one elongated flex member comprises an aperture configured for engaging with a protrusion of the scanhead.
20. The adapter according to item 19, wherein the width of the at least one elongated flex member is wider in the section comprising the aperture.
21. The adapter according to any of the preceding items, wherein the frame section forms a distal protrusion.
22. The adapter according to any of items 1-20, wherein the frame section forms a distal intrusion.
23. The adapter according to any of the preceding items, wherein the frame section forms a distal perimeter for contacting the skin of the subject.
24. The adapter according to item 23, wherein the perimeter has a shape selected from the group of a: circle, oval, triangular, square, rectangle, rhombus, trapezium, parallelogram, and polygons with more than four sides.
25. The adapter according to any of the preceding items, wherein the frame section forms a smooth cone for contacting the skin of the subject.
26. The adapter according to any of the preceding items, wherein the height of the frame section is between 0.5-10 mm, more preferably between 1-8 mm, and most preferably between 1.5-5 mm, such as 2 mm.
27. The adapter according to any of items 3-26, wherein the height of the frame section is identical to the chamber wall thickness.
28. The adapter according to any of the preceding items, wherein the width of the frame section is between 0.5-10 mm, more preferably between 1-5 mm, and most preferably between 1-2 mm, such as 1.2 mm.
29. The adapter according to any of the preceding items, wherein the frame section forms a distal opening to the cavity.
30. The adapter according to any of the preceding items, wherein the cavity is configured to be loaded with a fluid from the distal end.
31. The adapter according to any of the preceding items, comprising a polymeric material selected from the group of: polycarbonates, polystyrenes, polyamides, polyurethanes, polyethylenes, and copolymers, such as ABS copolymers.
32. The adapter according to item 31, comprising a polymeric material having a notched impact strength at 23° of between 10-30 kJ/m², more preferably between 12-25 kJ/m², and most preferably between 14-20 kJ/m², such as 15 or 18 kJ/m², and/or an impact strength at 23° of between 80-130 kJ/m², more preferably between 90-120 kJ/m², and most preferably between 100-120 kJ/m², such as 110 kJ/m².
33. The adapter according to any of items 31-32, comprising a polymeric material having a tensile modulus of between 2000-3000 MPa, more preferably between 2200-2800 MPa, and most preferably between 2300-2600 MPa, such as 2400 or 2500 MPa.
34. The adapter according to any of items 31-33, comprising a polymeric material having a yield stress of between 20-80 MPa, more preferably between 30-70 MPa, and most preferably between 40-60 MPa, such as 46, 50, 54 MPa.
35. The adapter according to any of items 31-34, comprising a polymeric material having a yield strain of between 0.5-10%, more preferably between 1-7%, and most preferably between 1.5-5%, such as 2, 2.6, or 3%.
36. The adapter according to any of items 31-35, comprising a polymeric material having a flexural strength at 23° of between 40-200 MPa, more preferably between 50-150 MPa, and most preferably between 60-100 MPa, such as 80 MPa.
37. The adapter according to any of the preceding items obtained by injection molding.
38. The adapter according to any of the preceding items, wherein the adapter is disposable and configured for single-use or configured for recycling.
39. An ultrasound scanhead comprising the adapter according to any of items 1-38.
40. Use of the ultrasound scanhead adapter according to any of items 1-38 or the ultrasound scanhead of item 39 for ultrasound skin imaging.

## Claims

1. An ultrasound scanhead adapter for attachment to an ultrasound scanhead, the adapter comprising
- a distal end having a frame section for contacting the skin of a subject, a height of the frame section defining
i. a cavity, and
ii. a fixed distance between the skin surface of the subject and a top of the ultrasound scanhead during scanning, and
- a proximal end forming a reversible snap-fit connection to the scanhead.

2. The adapter according to claim 1, wherein the proximal end is configured for partially enclosing at least a part of the scanhead.

3. The adapter according to any of the preceding claims, wherein the proximal end forms a chamber having a chamber wall and a proximal opening adapted for slidingly receiving the scanhead, preferably wherein the proximal end forms a dome shaped chamber.

4. The adapter according to claim 3, wherein the chamber proximal opening is adapted to be elastically dilated.

5. The adapter according to any of claims 3-4, wherein the chamber wall has a thickness of between 0.2-4 mm, more preferably between 0.4-3 mm, and most preferably between 0.5-2 mm, such as 1.8 mm.

6. The adapter according to any of claims 3-5, wherein the chamber wall comprises one or more slits extending from the proximal opening towards the distal end.

7. The adapter according to any of claims 3-6, wherein the chamber wall comprises two or more slits in parallel thereby forming an elongated flex member.

8. The adapter according to claim 7, wherein the proximal end of the elongated flex member is configured for elastically snap-fitting over at least a part of the scanhead, and/or wherein the proximal end of the elongated flex member comprises a concave flange.

9. The adapter according to any of claims 7-8, wherein the elongated flex members extend proximally to the proximal opening of the chamber.

10. The adapter according to any of claims 7-9, wherein the at least one elongated flex member forms an audible snap-fit to the scanhead.

11. The adapter according to any of the preceding claims, wherein the frame section forms a distal perimeter for contacting the skin of the subject.

12. The adapter according to any of the preceding claims, comprising a polymeric material selected from the group of: polycarbonates, polystyrenes, polyamides, polyurethanes, polyethylenes, and copolymers, such as ABS copolymers.

13. The adapter according to claim 12, comprising a polymeric material having a notched impact strength at 23° of between 10-30 kJ/m², more preferably between 12-25 kJ/m², and most preferably between 14-20 kJ/m², such as 15 or 18 kJ/m², and/or an impact strength at 23° of between 80-130 kJ/m², more preferably between 90-120 kJ/m², and most preferably between 100-120 kJ/m², such as 110 kJ/m².

14. The adapter according to any of the preceding claims, wherein the adapter is disposable and configured for single-use or configured for recycling.

15. Use of the ultrasound scanhead adapter according to any of claims 1-14 for ultrasound skin imaging.
